# EUROPEAN PATENT APPLICATION

(11) **EP 1 854 447 A1**
(43) Date of publication of application: **14.11.2007**
(21) Application number: 06713820.6
(22) Date of filing: 09.02.2006
(51) Int. Cl.: A61K 6/083, C08F 20/26, C08K 3/00, C08L 33/14, C08L 101/00

(54) **DENTAL RESTORATION MATERIAL COMPOSITION**

(30) Priority: 10.02.2005 JP 2005035024
(71) Applicant: SUN MEDICAL CO., LTD., Shiga-ken 524-0044 (JP)
(72) Inventor: SAIMI, Yasukazu, riyama-shi, Shiga, 5240044 (JP); IMAI, Hirofumi, riyama-shi, Shiga, 5240044 (JP)
(74) Representative: Albrecht, Thomas
(86) International application number: PCT/JP2006/302679
(87) International publication number: WO 2006/085682

(57) **Abstract**

A dental restorative composition comprising a polymerizable monomer having at least 3 partial chains which are polyester chains bonded to polymerizable groups in one molecule. The dental restorative composition having high mechanical properties, especially high tenacity and excellent impact resistance and fracture resistance as a resin prosthetic material such as a resin for dental crows and artificial teeth, a filler material such as a composite resin, or other resin-based dental material is provided.

## Description

### TECHNICAL FIELD

The present invention relates to a dental restorative composition. More specifically, it relates to a novel dental restorative composition which has excellent mechanical properties, especially tenacity, and can be advantageously used as a general dental resin-based material exemplified by resin-based prosthetic materials such as resins for dental crowns, artificial tooth and resin inlays, resin-based filler materials such as composite resins, and Fischer sealants, dental surface coating materials and cavity coating materials, and a dental restorative composition containing powders of a cured product of the above dental restorative composition.

### BACKGROUND ART

Nowadays, in the field of dental care, resin-based dental materials such as resin-based prosthetic materials including resins for dental crowns, resin inlays, resin unlays, artificial tooth and dentures, resin-based filler materials such as composite resins, Fischer sealants for closing small lacunae and fissures, and resin-based coating materials for protection after the formation of cavities are often used for dental restorations for deficit tooth in place of inorganic and metal-based dental materials. One of the resin-based dental materials is a paste prepared by mixing a resin-based material which is often used in the prosthesis and preservation fields with a polymerizable monomer, inorganic filler powders and a polymerization initiator. The characteristic properties of a cured product of the paste are often determined by the filling rate and average particle diameter of the filler powders. For example, a paste prepared by using glass powders having an average particle diameter of about 100 µm as a filler has a disadvantage that its polishing performance is low due to the huge particle diameter of the glass powders, thereby making it difficult to obtain a lustrous surface though its mechanical strength is high, and a problem that a resin portion on the surface of the cured product is selectively worn away in the cavity of a mouth and glass powders project to become like a file, thereby causing the abrasion of the opposing tooth or the dental material.

In order to solve the above problem, JP-A 62-89701, JP-B 62-86003, JP-B 1-57082 and US Patent No. 4764497 propose a method using a spherical inorganic filler having a particle diameter of 0.1 to 1 µm and a uniform particle size distribution. As this inorganic filler is spherical and uniform in size, it can be mixed with a polymerizable monomer in a large amount and provides high mechanical strength and excellent surface gloss. However, as spherical inorganic filler powders are used, the effect of anchoring them in a resin is lower than that for amorphous inorganic filler powders and they easily fall off from the resin.

There is also proposed a method using a super fine inorganic filler having a particle diameter of 0.1 µm or less as an inorganic filler. When the powders of the filler are used, the surface becomes lustrous and the opposing object is not worn away. However, when it is mixed in a large amount, the viscosity of the obtained paste rises due to the large surface area of the inorganic filler and when the amount of the filler is reduced, the paste becomes sticky and adheres to a spatula, thereby causing an operation problem such as the reduced operation ease of the paste and a defect such as low mechanical strength.

Then, in JP-A 56-20066, attempts are made to solve the above problems by using a filler (to be referred to as "composite filler" hereinafter) obtained by coating inorganic filler powders having a diameter of 0.1 µm or less with a polymerizable monomer and polymerizing the monomer. Since the composite filler manufactured by this method comprises a polyfunctional (meth)acrylate compound having 3 or more ethylenically unsaturated groups in a (meth)acrylate-based polymerizable monomer, all the ethylenically unsaturated groups are not polymerized and some of them remain. A cured product of a dental restorative material comprising this composite filler has improved mechanical strength because the ethylenically unsaturated groups remaining on the surface of the composite filler and the polymerizable monomer constituting a matrix are covalently bonded together. Further, the dental restorative material has excellent abrasion resistance because the composite filler does not fall off. However, as the composite filler itself is very rigid and brittle, its cured product is easily broken and inferior in impact resistance.

To improve this brittleness, JP-A 60-71621 discloses a composite filler powder obtained by kneading an adduct of hydroxyl alkyl(meth)acrylate and diisocyanate in a weight ratio of 2:1 or a mixture of polyethylene glycol di(meth)acrylate and an inorganic filler powder with an acrylic polymerizable monomer having 3 or more ethylenically unsaturated groups in the molecule, polymerizing them and grinding the resulting polymer. Although the composite filler powder disclosed by this patent has lower brittleness than that of the composite filler powder disclosed by JP-A 56-20066, its effect is still unsatisfactory.

Although a dental composition comprising a polymerizable monomer having an urethane bond (refer to JP-A 57-85355 and JP-A 5-262615), a dental composition comprising a polyethylenically unsaturated carbamoyl isocyanurate-based monomer and a composite filler, and a dental composition comprising this composite filler (refer to JP-A 7-80736 and JP-A 5-246819) have excellent mechanical strength, discoloration or deterioration may occur by heat or light. Since a cured product of a dental composition comprising a dipentaerythritol (meth)acrylate-based monomer (refer to JP-A 57-35505 and JP-A 63-183904) is rigid and brittle, when it is used as a dental restorative material, it is hard to say that its mechanical properties such as fracture resistance are satisfactory.

Some of the polymerizable compounds having 3 or more ester chains bonded to a polymerizable group in one molecule described in the present invention are disclosed by JP-A 6-16799. This publication teaches that there is provided a material capable of ON-OFF control of releasing a chemical at any temperature range by controlling the heat transition temperature by changing the structure of an aliphatic polyester chain or the average degree of polymerization of the polymer. The publication fails to disclose the application of this polymerizable compound in the dental restorative composition of the present invention. Therefore, a resin-based restorative material having excellent mechanical properties, especially high tenacity as well as excellent impact resistance and fracture resistance is still desired.

### DISCLOSURE OF THE INVENTION

It is an object of the present invention to provide a dental restorative composition which can be advantageously used as a general dental resin-based material exemplified by resin-based prosthetic materials such as resins for dental crowns, artificial tooth and resin inlays, resin-based filler materials such as composite resins, and Fischer sealants and cavity coating materials and which has excellent mechanical properties, especially high tenacity as well as excellent impact resistance and fracture resistance.

According to the present invention, the above object and advantage of the present invention can be attained by a dental restorative composition comprising a polyfunctional polymerizable compound having at least 3 partial chains in one molecule, each having a polymerizable group, an ester bond and a length of at least 7 atoms.

Other objects and advantages of the present invention will become apparent from the following description.

### BEST MODE FOR CARRYING OUT THE INVENTION

A description is first given of the polyfunctional polymerizable compound (may be referred to as "polyfunctional polymerizable compound (A)" hereinafter) used in the present invention. This polyfunctional polymerizable compound has a at least 3 partial chains in one molecule, each having a polymerizable group, an ester bond and a length of 7 atoms or more.

The polymerizable group is preferably a radically polymerizable group such as an ethylenically unsaturated bond.

The at least 3 partial chains are independent from one another and have a length of 7 atoms or more, preferably 9 atoms or more, more preferably 11 atoms or more. When the number of atoms is smaller than the above lower limit value, impact resistance and fracture resistance degrade disadvantageously. The upper limit value is not particularly limited. However, if the partial chains are too long, mechanical strength lowers. Therefore, the upper limit value is preferably 100 atoms or less, more preferably 50 atoms or less, much more preferably 30 atoms or less.

The chain length of the partial chain represents the number of atoms on the main chain of the partial chain. When the oxygen atoms of an ether are existent on the main chain, the number of the oxygen atoms of the ether is not included in the number of atoms on the main chain. As for the ring structure in the chain, a longer partial ring structure is adopted. When it is complicated such as a branch structure, a main chain is selected so that the polymerizable group and the ester bond are situated on the main chain.

The oxygen atoms (-O-) of an ester (R-CO-O-R, R is an alkyl, the same shall apply hereinafter) have low polarity and low hydrophilic nature advantageously whereas the oxygen atom (-O-) of an ether (R-O-R) has high polarity and high hydrophilic nature disadvantageously. Therefore, the average number of the oxygen atoms of an ether on one partial chain is preferably 5 or less, more preferably 3 or less, much more preferably 1 or less. The ratio of the number of the oxygen atoms of an ether to the number of other atoms is preferably 0.3 or less, more preferably 0.2 or less, much more preferably 0.05 or less.

The particle chain will be described, taking the following polyfunctional polymerizable compound as an example.

W(̵V-V-V-COO-CH=CH2)₃

wherein V is a group of divalent atoms including one atom constituting the main chain and W is a group of trivalent atoms including one atom constituting the main chain.

In the above compound, as the moiety -V-V-V-COO-CH=CH₂ has a polymerizable group (-CH=CH₂), an ester bond (-COO-) and a chain length of 7 atoms, it is understood that it corresponds to the partial chain in the present invention. Since 3 partial chains are separated from one another by the atomic group W, the three partial chains are existent in one molecule.

In contrast to this, in the case of the following example

W(̵V-V-COO-CH=CH₂)₃

wherein W and V are as defined above, as the moiety V-V-COO-CH=CH₂ has a polymerizable group and an ester bond but a chain length of 6 atoms, it does not correspond to the partial chain in the present invention. Since -W-V-V-COO-CH=CH₂ obtained by adding W to the above moiety has a chain length of 7 atoms, it corresponds to the partial chain in the present invention. In this case, as one partial chain having a length of 7 atoms and two partial chains having a length of 6 atoms are existent in the molecule, the compound of this example does not correspond to the polyfunctional compound in the present invention.

If such conditions as the chain length and the functional group are satisfied, dividing a long linear molecular chain arbitrarily to define a plurality of the partial chains of the present invention is against the subject matter of the present invention. At least a branch point where the another molecular chain of the present invention is branched should be defined as the end point of the molecular chain of the present invention.

It is preferred that the polymerizable functional group should be situated on a molecular end side rather than the main chain side of the molecule in the partial chain of the molecule of the compound (A) of the present invention because crosslinking is easily effected at high efficiency.

An atomic group which may be existent on the chain other than the ester bond (-CO-O-) in the partial chain is not particularly limited if it is an atomic group having high hydrophilic nature such as the crosslinking of the oxygen of an ether as described above or does not impede the effect of the present invention such as an atomic group having toxicity to the human body. Examples of the atomic group include a hydrocarbon group, sulfonic acid residue and phosphoric acid residue. Out of these, a hydrocarbon group is preferred. More specifically, the partial chain preferably has a polyester structure.

Examples of the polyester chain include polyester chains such as aliphatic polyester chains, aromatic ring-containing polyester groups, hetero ring-containing polyester groups, carbonate group-containing polyester groups and alkylene glycol group-containing polyester groups. At least one polymerizable group should be bonded to one terminal and/or side chain of the polyester chain, preferably to one terminal of the polyester chain. Different polyester chains, for example, an aliphatic polyester chain and an aromatic ring-containing polyester chain may be bonded in one molecule at the same time. The polymerizable group is preferably a radically polymerizable group having an ethylenically unsaturated bond such as a vinyl group or vinylidene group, more preferably a (meth)acryloyl group.

The manufacturing method and type of the above polyfunctional compound in the present invention are not particularly limited, and a preferred manufacturing method and type may be employed. The method disclosed by, for example, JP-A 6-16799 may be adopted. As a specific preferred manufacturing method, a polyhydric alcohol compound having 3 or more hydroxyl groups in one molecule such as a triol compound, tetraol compound or hexaol compound and one or more cyclic ester compounds are mixed and reacted with each other, and further the residual hydroxyl group and a carboxylic acid having a polymerizable group or an acid halide thereof are reached with each other in the presence of a catalyst such as a tin-based compound to manufacture the above polyfunctional compound.

Examples of the polyhydric alcohol compound include glycerin, 1,1,1-tri(hydroxymethyl)ethane, 1,1,1-tri(hydroxymethyl)propane, 1,1,1-tri(hydroxymethyl)pentane, 1,1,1-tri(hydroxymethyl)hexane, 1,1,1-tri(hydroxymethyl)heptane, pentaerythritol, 1,3,5-tri(hydroxymethyl)pentane, 1,3,3,5-tetra(hydroxymethyl)pentane, 1,2,6-trihydroxyhexane, 1,2,6-tetrahydroxyhexane, pentaerythritol and dipentaerythritol.

Examples of the cyclic ester compound include glycolide, D,L-lactide, L-lactide, D-Iactide, β-butyrolactone, γ-butyrolactone, β-valerolactone, γ-valerolactone, δ-valerolactone, δ-caprolactone, γ-caprolactone and ε-caprolactone.

The preferred structure of the above polyfunctional compound will be described more specifically.

First, the polyfunctional compound preferably has the structure (following formula (I)) of a partial chain which is a polyester chain bonded to a polymerizable group.

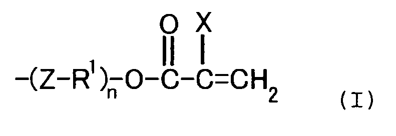

In the above formula, Z is -OCO- or -COO-, and R¹ is an alkylene group having 1 to 10 carbon atoms, divalent group having an aromatic ring and/or a hetero ring, preferably a linear or branched alkylene group having 1 to 5 carbon atoms, or divalent group having an aromatic ring and/or a hetero ring, more preferably a linear alkylene group having 1 to 3 carbon atoms. X is a hydrogen atom, halogen atom, cyano group, linear or branched alkyl group having 1 to 10 carbon atoms, or monovalent group having an aromatic ring and/or a hetero ring, preferably a hydrogen atom, or linear or branched alkyl group having 1 to 5 carbon atoms, more preferably a hydrogen atom or methyl group and n is an integer of 1 to 10, preferably 1 to 8, more preferably 1 to 6.

The partial chain which is a polyester chain bonded to a polymerizable group preferably has a structure represented by the following formula (Ia).

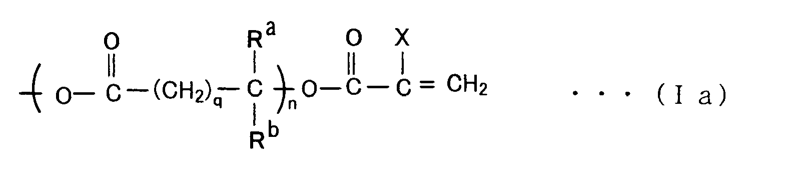

In the above formula, R^{a} and R^{b} are each independently a hydrogen atom, linear or branched alkyl group having 1 to 10 carbon atoms or monovalent group having an aromatic ring and/or a hetero ring, preferably a hydrogen atom, linear or branched alkyl group having 1 to 5 carbon atoms or monovalent group having an aromatic ring and/or a hetero ring, more preferably a hydrogen atom or linear alkyl group having 1 to 3 carbon atoms, q is an integer of 0 to 10, preferably 1 to 10, more preferably 1 to 8, and X and n are as defined in the formula (I).

If the methylene group in the above formula (Ia) does not impair the mechanical properties of the present invention, it may be a group substituted by a propylene group or an alkylene group other than methylene group such as a group having both methylene group and propylene group, or a group substituted by an oxyalkylene group such as oxymethylene group or oxyethylene group, polyoxyalkylene group, polyalkylene carbonate group or group other than these.

The polyfunctional compound is preferably at least one selected from the group consisting of compounds represented by the following formula (II) and (III).

R³ₘ-C((CH₂)ₚ-R²)₄₋ₘ (II)

In the above formula, R² is a partial chain represented by the above formula (I), R³ is a hydrogen atom, linear or branched alkyl group having 1 to 10 carbon atoms, group having an aromatic ring and/or a hetero ring or group having a polymerizable group, preferably a hydrogen atom, linear or branched alkyl group having 1 to 5 carbon atoms or group having a polymerizable group, more preferably a hydrogen atom, linear alkyl group having 1 to 3 carbon atoms or group having a polymerizable group, p is an integer of 0 to 10, preferably 1 to 10, more preferably 1 to 8, and m is 0 or 1. A (4-m) number of groups represented by the following formula may be the same or different.

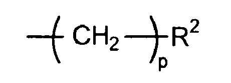

Since the polyfunctional polymerizable compound represented by the formula (II) can be made homogeneous with various polymerizable monomers, its composition can be adjusted according to its purpose. When this polyfunctional polymerizable compound is used, the mechanical properties, especially tenacity of a cured product of the dental restorative composition are improved, thereby making it most suitable for the prevention of breakage or chipping of a resin-based prosthetic material or a resin-based restorative material.

Further, a compound represented by the following formula (III) is also preferred as the above polyfunctional polymerizable compound.

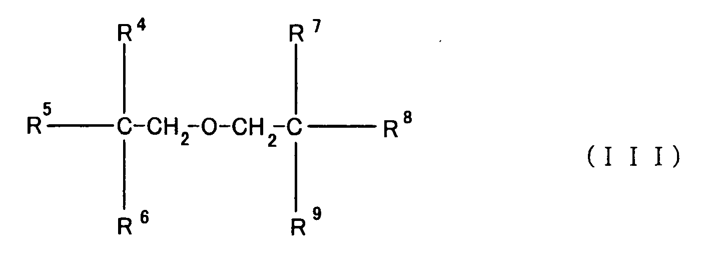

In the above formula, R⁴, R⁵, R⁶, R⁷, R⁸ and R⁹ are each independently a group represented by the following formula: 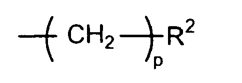 hydroxyl group, linear or branched alkyl group having 1 to 10 carbon atoms, linear or branched hydroxyalkyl group having 1 to 10 carbon atoms, group having an aromatic ring and/or a hetero ring, group having a polymerizable group different from a group represented by the following formula: 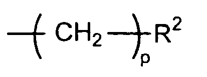 with the proviso that at least three out of R⁴ to R⁹ are groups represented by the following formula: 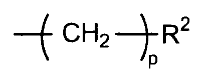 and R² and p are as defined in the above formula (II).

The group represented by the following formula is preferably a polyester group bonded to a polymerizable group, 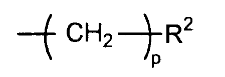 more preferably a group represented by the above formula (I), much more preferably a group represented by the above formula (Ia), particularly preferably a group represented by the following formula (IV).

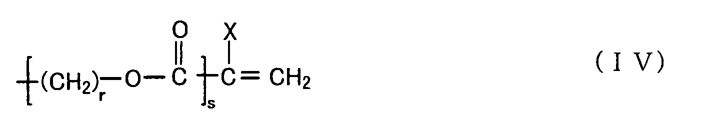

In the above formula (IV), r and s are each independently an integer of 0 to 10, preferably 1 to 8, more preferably 1 to 5. X is as defined in the above formula (I) .

When the polyfunctional polymerizable compound represented by the above formula (III) is used, the mechanical properties, especially tenacity of a cured product of the dental restorative composition is improved, thereby making it most suitable for the prevention of breakage or chipping of a resin-based prosthetic material or a resin-based restorative material. To further improve the mechanical properties by increasing the polymerization ratio with the resin component of the dental restorative composition, R⁴ to R⁹ in the above formula (III) are more preferably groups represented by the above formula (Ia) or (IV) rather than groups having no reactive group. When the number of the groups of the above formula (Ia) is 3 or more, preferably 4 or more, much more preferably 6, the polymerization ratio with the resin component is improved and the number of the polyester chains contributing to the development of tenacity in one molecule becomes large, whereby a cured product having high tenacity is obtained.

The composition of the present invention may contain another polymerizable compound besides the above polyfunctional polymerizable compound.

A known polymerizable compound may be used as the another polymerizable compound.

Examples of the known polymerizable compound include (i) monofunctional polymerizable monomers, (ii) bifunctional polymerizable monomers, (iii) trifunctional polymerizable monomers, and (iv) polymerizable monomers having a functionality of 4 or more, out of which (meth)acrylate compounds are particularly preferred. Examples of these monomers are given below.
(i) monofunctional polymerizable monomers
   The monofunctional polymerizable monomers (i) include (meth) acrylate-based polymerizable monomers such as methyl (meth)acrylate, ethyl (meth)acrylate, n-butyl (meth)acrylate, isopropyl (meth)acrylate, 2-ethylhexyl (meth)acrylate, n-lauryl (meth)acrylate, n-stearyl (meth)acrylate, behenyl(meth)acrylate, 2-hydroxyethyl (meth)acrylate, 2-hydroxy-3-phenoxypropyl (meth)acrylate, tetrafurfuryl (meth)acrylate, glycidyl (meth)acrylate, methoxyethylene glycol (meth)acrylate, methoxydiethylene glycol (meth)acrylate, methoxytriethylene glycol (meth) acrylate, methoxypolyethylene glycol (meth) acrylate, ethoxyethylene glycol (meth)acrylate, ethoxydiethylene glycol (meth)acrylate, ethoxytriethylene glycol (meth)acrylate, ethoxypolyethylene glycol (meth)acrylate, phenoxyethylene glycol (meth)acrylate, phenoxydiethylene glycol (meth)acrylate, phenoxytriethylene glycol (meth) acrylate, phenoxypolyethylene glycol (meth)acrylate, cyclohexyl (meth)acrylate, benzyl (meth)acrylate, 3-chloro-2-hydroxypropyl (meth)acrylate, isobornyl (meth)acrylate, dicyclopentanyl (meth)acrylate, dicyclopentenyl (meth)acrylate, dicyclopentenyloxyethyl (meth)acrylate, N-(meth)acryloylmorpholine, trifluoroethyl (meth)acrylate, perfluorooctyl (meth)acrylate,y-(meth)acryloyloxypropyl trimethoxysilane and γ-(meth)acryloyloxypropylmethyl dimethoxysilane. Polymerizable monomers having a polyester chain bonded to one polymerizable group and polymerizable monomers having one (meth) acrylate group and a polycarbonate group may also be used.
   Known acid group-containing polymerizable monomers may also be used. The monomers include phosphoric acid group-containing polymerizable monomers, pyrophosphoric acid group-containing polymerizable monomers, thiophosphoric acid group-containing polymerizable monomers, carboxylic acid group-containing polymerizable monomers and sulfonic acid group-containing polymerizable monomers.
   Out of these, the phosphoric acid group-containing polymerizable monomers include a polymerizable monomer such as 2-(meth)acryloyloxyethyldihydrogen phosphate, 3-(meth)acryloyloxypropyldihydrogen phosphate, 10-(meth)acryloyloxydecyldihydrogen phosphate, 12-(meth)acryloyloxydodecyldihydrogen phosphate, (meth)acryloyloxyethylphenyl phosphate and (8-(meth)acryloyloxy)octyl-3-phosphonopropinate.
   The pyrophosphoric acid group-containing polymerizable monomers include a polymerizable monomer such as di[2-(meth)acryloyloxyethyl]pyrophosphate, di [4-(meth)acryloyloxybutyl]pyrophosphate, di[8-(meth)acryloyloxyoctyl]pyrophosphate and di[12-(meth)acryloyloxydodecyl]pyrophosphate.
   The thiophosphoric acid group-containing polymerizable monomers include a polymerizable monomer such as 2-(meth)acryloyloxyethyldihydrogen thiophosphate, 3-(meth)acryloyloxypropyldihydrogen thiophosphate, 10-(meth)acryloyloxydecyldihydrogen thiophosphate and 12-(meth)acryloyloxydodecyldihydrogen thiophosphate.
   The carboxylic acid group-containing polymerizable monomers include a polymerizable monomer such as (meth)acrylic acid, 2-(meth)acryloyloxyethyloxycarbonylphthalic acid, 4-(meth)acryloyloxybutyloxycarbonylphthalic acid, 8-(meth)acryloyloxyoctyloxycarbonylphthalic acid, 10-(meth)acryloyloxydecyloxycarbonylphthalic acid, acid anhydrides thereof, 6-(meth)acryloylaminohexylcarboxylic acid, 8-(meth)acryloylaminooctylcarboxylic acid and 11-(meth)acryloyloxy-1,1-undecanedicarboxylic acid.
   The sulfonic acid group-containing polymerizable monomers include a polymerizable monomer such as 2-(meth)acrylamideethylsulfonic acid, 3-(meth)acrylamidepropylsulfonic acid, 4-(meth)acrylamidebutylsulfonic acid and 10-(meth)acrylamidedecylsulfonic acid.
   Out of these monofunctional polymerizable monomers (i), methyl (meth)acrylate, ethyl (meth)acrylate, n-butyl (meth)acrylate, isopropyl (meth)acrylate, tetrafurfuryl (meth)acrylate and isobornyl (meth)acrylate are preferred.
(ii) bifunctional polymerizable monomers
   The bifunctional polymerizable monomers (ii) include aromatic polymerizable compounds such as 2,2-bis((meth)acryloyloxyphenyl)propane, 2,2-bis[4-(3-(meth)acryloyloxy)-2-hydroxypropoxyphenyl] propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth) acryloyloxydiethoxyphenyl)propane, 2-(4-(meth)acryloyloxydiethoxyphenyl)-2-(4-(meth) acryloyloxyditriethoxyphenyl)propane, 2-(4-(meth) acryloyloxydipropoxyphenyl)-2-(4-(meth) acryloyloxytriethoxyphenyl)propane, 2,2-bis(4-(meth)acryloyloxydipropoxyphenyl)propane and 2,2-bis(4-(meth)acryloyloxyisopropoxyphenyl)propane; and diadducts obtained by the addition polymerization of a hydroxyl group-containing (meth)acrylate compound corresponding to each of the above (meth)acrylates such as 2-hydroxyethyl (meth)acrylate, 2-hydroxyethylpropyl (meth)acrylate or 3-chloro-2-hydroxypropyl (meth)acrylate and a diisocyanate compound having an aromatic group such as diisocyanate methyl benzene or 4,4'-diphenylmethane diisocyanate.
   Aliphatic compounds include ethylene glycol-based and propylene glycol-based di(meth)acrylates such as ethylene glycol di(meth)acrylate, diethylene glycol di(meth)acrylate, triethylene glycol di(meth)acrylate, tetraethylene glycol di(meth)acrylate, pentamethylene glycol di(meth)acrylate, hexaethylene glycol di(meth)acrylate, nonaethylene glycol di(meth)acrylate, propylene glycol di(meth)acrylate, dipropylene glycol di(meth)acrylate, tripropylene glycol di(meth)acrylate, tetrapropylene glycol di(meth)acrylate, pentapropylene glycol di(meth)acrylate, hexapropylene glycol di (meth) acrylate and nonapropylene glycol di (meth) acrylate, di(meth)acrylate compounds having a cyclic, linear or branched aliphatic group bonded to ethylene glycol or propylene glycol, such as ethoxylated cyclohexane di(meth)acrylate, aliphatic di(meth)acrylate compounds such as neopentyl glycol di(meth)acrylate, tricyclodecane dimethanol di(meth)acrylate, 1,4-butanediol di(meth)acrylate, 1,6-hexanediol di(meth)acrylate, 1,9-nonanediol di(meth)acrylate and 1,12-dodecanediol di(meth)acrylate, adducts obtained by the addition polymerization of a hydroxyl group-containing (meth) acrylate compound corresponding to each of the above (meth)acrylates such as 2-hydroxyethyl (meth)acrylate, 2-hydroxyethylpropyl (meth)acrylate or 3-chloro-2-hydroxypropyl (meth)acrylate and a diisocyanate compound such as hexamethyl diisocyanate, trimethylhexamethylene diisocyanate, diisocyanate methyl cyclohexane, isophorone diisocyanate or methylenebis(4-cyclohexylisocyanate), and di(2-(meth)acryloyloxypropyl)phosphate. Compounds having two (meth) acrylate groups and a polyester group, for example, polymerizable monomers having two (meth)acrylate groups bonded to Praccel 200 series (polycaprolactone diol, manufactured by Daicel Chemical Industries, Ltd.) may also be used. Further, compounds having two (meth)acrylate groups and a polycarbonate group, for example, polymerizable monomers having two (meth) acrylate groups bonded to Praccel CD (polycarbonate diol) may also be used.
   Out of the bifunctional polymerizable monomers (ii), 2,2-bis((meth)acryloyloxyphenyl)propane, 2,2-bis[4-(3-(meth)acryloyloxy)-2-hydroxypropoxyphenyl] propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, adducts obtained by the addition polymerization of a hydroxyl group-containing (meth)acrylate compound corresponding to each of the above (meth)acrylates, such as 2-hydroxyethyl (meth)acrylate, 2-hydroxyethylpropyl (meth)acrylate or 3-chloro-2-hydroxypropyl (meth)acrylate and a diisocyanate compound such as hexamethyl diisocyanate, trimethylhexamethylene diisocyanate, diisocyanate methyl cyclohexane, isophorone diisocyanate or methylenebis(4-cyclohexylisocyanate), di(2-(meth)acryloyloxypropyl)phosphate, ethylene glycol di(meth)acrylate, diethylene glycol di(meth)acrylate, triethylene glycol di(meth)acrylate and tetraethylene glycol di(meth)acrylate are preferred.
(iii) trifunctional polymerizable monomers
   The trifunctional polymerizable monomers (iii) include trimethylolpropane tri(meth)acrylate, trimethylolethane tri(meth)acrylate, pentaerythritol tri(meth)acrylate, dipentaerythritol tri(meth)acrylate, ethoxylated trimethylolpropane tri(meth)acrylate, propoxylated trimethylolpropane tri(meth)acrylate and tris(2-(meth)acryloxyethyl)isocyanurate. Compounds having 3 (meth)acrylate groups and a polyester group, for example, polymerizable monomers having 3 (meth)acrylate groups bonded to Praccel 300 series (polycaprolactone diol, manufactured by Daicel Chemical Industries, Ltd.), which differ from the above polyfunctional polymerizable compound used as the essential component in the present invention, may also be used. Polymerizable monomers having 3 (meth)acrylate groups and a polycarbonate group may also be used.
   Out of the trifunctional polymerizable monomers (iii), trimethylolpropane tri(meth)acrylate, ethoxylated trimethylolpropane tri(meth)acrylate, tris(2-(meth)acryloxyethyl isocyanurate are preferred. Polymerizable monomers having 3 (meth)acrylate groups and a polyester group and polymerizable monomers having 3 (meth)acrylate groups and a polycarbonate group which differ from the above polyfunctional polymerizable compound used as the essential component in the present invention may also be used.
(iv) polymerizable monomers having a functionality of 4 or more
   The polymerizable monomers having a functionality of 4 or more (iv) include tetra(meth)acrylate compounds such as pentaerythritol tetra(meth)acrylate, ethoxylated pentaerythritol tetra(meth)acrylate, propoxylated pentaerythritol tetra(meth)acrylate, dipentaerythritol tetra(meth)acrylate, ethoxylated dipentaerythritol tetra(meth)acrylate, propoxylated dipentaerythritol tetra(meth)acrylate, ditrimethylolpropane tetra(meth)acrylate and ethoxylated ditrimethylolpropane tetra(meth)acrylate, diisocyanate compounds having an aliphatic group between diisocyanates such as hexamethyl diisocyanate, trimethylhexamethylene diisocyanate, diisocyanate methyl cyclohexane, isophorone diisocyanate and methylenebis(4-cyclohexylisocyanate), adducts obtained by the addition polymerization of a diisocyanate compound having an aromatic group such as diisocyanate methyl benzene or 4,4'-diphenylmethane diisocyanate and glycidol di(meth)acrylate, dipentaerythritol penta(meth)acrylate and dipentaerythritol hexa(meth)acrylate. Polyethylenically unsaturated carbamoyl isocyanurate compounds disclosed by JP-B 7-80736 may also be used. Polymerizable monomers having 4 or more (meth)acrylate groups and a polyester group and polymerizable monomers having 4 or more (meth) acrylate groups and a polycarbonate group which differ from the above polyfunctional polymerizable compound used as the essential component in the present invention may also be used.

Out of the polymerizable monomers having a functionality of 4 or more (iv), ditrimethylolpropane tetra (meth) acrylate, dipentaerythritol hexa(meth)acrylate and the above polyethylenically unsaturated carbamoyl isocyanurate-based compounds are preferred.

When the above polyfunctional polymerizable compound containing a polyester group (A) and the above another polymerizable compound are used in combination, the weight ratio of these compounds is not limited and may be suitably determined to develop the performance of the present invention. To enable a cured product of the dental restorative composition to form a structure having tenacity, the weight ratio of the former to the latter is 99 to 1:1 to 99, preferably 95 to 5: 5 to 95, more preferably 90 to 10:10 to 90.

The composition of the present invention preferably contains a polymerization initiator. A description is subsequently given of the polymerization initiator (may be referred to as "polymerization initiator (B) " hereinafter). A known polymerization initiator such as a photopolymerization initiator (B1), thermopolymerization initiator (B2) or redox initiator (B3) may be used as the polymerization initiator (B).

An optical sensitizer alone or a combination of an optical sensitizer and a photopolymerization accelerator may be used as the photopolymerization initiator (B1). Known compounds which are excited by ultraviolet light or visible light to initiate polymerization, such as α-diketone compounds including benzyl and camphorquinone, and α-naphthyl, p,p'-dimethoxybenzyl, pentadione, 1,4-phenanthrenequinone, naphthoquinone and diphenyltrimethylbenzoyl phosphine oxide may be used alone or in combination of two or more as the optical sensitizer. Out of these, camphorquinone, acylphosphine oxides such as diphenyltrimethylbenzoylphosphine oxide and derivatives thereof are particularly preferably used.

When the photopolymerization initiator (B1) is used, a photopolymerization accelerator is preferably used. Examples of the photopolymerization accelerator used herein include p-toluenesulfinic acid and alkali metal salts thereof; tertiary amines such as N,N-dimethylaniline, N,N-diethylaniline, N,N-dibenzylaniline, N,N-dimethyl-p-toluidine, p-N,N-dimethylaminobenzoic acid, p-N,N-diethylaminobenzoic acid, ethyl p-N,N-dimethylaminobenzoate, ethyl p-N,N-diethylaminobenzoate, methyl p-N,N-dimethylaminobenzoate, methyl p-N,N-diethylaminobenzoate, p-N,N-dimethylaminobenzaldehyde, 2-n-butoxyethyl p-N,N-dimethylaminobenzoate, 2-n-butoxyethyl p-N,N-diethylaminobenzoate, p-N,N-dimethylaminobenzonitrile, p-N,N-diethylaminobenzonitrile, p-N,N-dihydroxyethylaniline, p-dimethylaminophenethyl alcohol, N,N-dimethylaminoethyl methacrylate, triethylamine, tributylamine, tripropylamine and N-ethylethanolamine, and secondary amines such as N-phenylglycin and alkali metal salts of N-phenylglycin; a combination of the above tertiary amine or secondary amine and citric acid, malic acid or 2-hydroxypropanoic acid; barbituric acids such as 5-butylaminobarbituric acid and 1-benzyl-5-phenylbarbituric acid; and organic peroxides such as benzoyl peroxide and di-tert-butyl peroxide. These photopolymerization accelerators may be used alone or in combination of two or more. Out of these, tertiary aromatic amines having a nitrogen atom directly bonded to an aromatic group such as ethyl p-N,N-dimethylaminobenzoate, 2-n-butoxyethyl p-N,N-dimethylaminobenzoate and N,N-dimethylaminoethyl methacrylate, aliphatic tertiary amines having a polymerizable group such as N,N-dimethylaminoethyl methacrylate, and secondary amines such as N-phenylglycin and alkali metal salts of N-phenylglycin are particularly preferred. To complete the curing of the dental restorative composition of the present invention as quickly as possible, a combination of an optical sensitizer and a photopolymerization accelerator is preferably used. A combination of camphorquinone and (a) an ester compound of a tertiary aromatic amine having a nitrogen atom directly bonded to an aromatic group, such as ethyl p-N,N-dimethylaminobenzoate or 2-n-butoxyethyl p-N,N-dimethylaminobenzoate, an aliphatic tertiary amine having a polymerizable group such as N,N-dimethylaminoethyl methacrylate or a secondary amine such as N-phenylglycin or an alkali metal salt of N-phenylglycin; a combination of camphorquinone and (b) acylphosphine oxide; a combination of camphorquinone and (c) acylphosphine oxide and p-toluenesulfinic acid or alkali metal salt; a combination of camphorquinone and (d) an ester compound of a tertiary aromatic amine having a nitrogen atom directly bonded to an aromatic group and acylphosphine oxide; and a combination of camphorquinone and (e) an ester compound of a tertiary aromatic amine having a nitrogen atom directly bonded to an aromatic group and an alkali metal salt of p-toluenesulfinic acid are particularly preferably used. The amount of the polymerization accelerator is not particularly limited if optical curability is promoted but generally 1 to 200 parts by weight based on 100 parts by weight of the photopolymerization initiator.

An organic peroxide or a diazo compound is preferably used as the thermopolymerization initiator (B2). To carry out polymerization efficiently in a short period of time, a compound having a decomposition half life at 80°C of 10 hours or less is preferred. Examples of the organic peroxide include diacyl peroxides such as acetyl peroxide, isobutyl peroxide, decanoyl peroxide, benzoyl peroxide and succinic acid peroxide; peroxydicarbonates such as diisopropylperoxy dicarbonate, di-2-ethylhexylperoxy dicarbonate and diallylperoxy dicarbonate; peroxy esters such as tert-butylperoxy isobutyrate, tert-butyl neodecanate and cumeneperoxy neodecanate; and peroxide sulfonates such as acetylcyclohexylsulfonyl peroxide.

Examples of the diazo compound include 2,2'-azobisisobutyronitrile, 4,4'-azobis(4-cyanovaleric acid), 2,2'-azobis(4-methoxy-2,4-dimethoxyvaleronitrile) and 2,2'-azobis(2-cyclopropylpropionitrile). Benzoyl peroxide and 2,2'-azobisisobutyronitrile are particularly preferred.

A redox initiator (B3) which is a combination of an organic peroxide such as benzoyl peroxide and a reducing agent, for example, a tertiary amine such as N,N-dimethyl-p-toluidine and initiates polymerization at around normal temperature may also be used.

The photopolymerization initiator (B1), the thermopolymerization initiator (B2) and the redox initiator (B3) may be used in combination.

The ratio of the above polyfunctional polymerizable compound (A) to the polymerization initiator (B) may be suitably determined in consideration of the mechanical properties of a cured product of the dental restorative composition and the color before and after curing. The amount of the component (B) is preferably 0.005 to 10 parts by weight, more preferably 0.01 to 5 parts by weight, much more preferably 0.01 to 3 parts by weight based on 100 parts by weight of the component (A).

Preferably, the composition of the present invention comprises a filler. A description is subsequently given of the filler (C). An inorganic filler (C1) or an organic filler (C2) is used as the filler. A composite filler (D) which is an organic and inorganic composite filler may be used without restriction. The composite filler (D) will be described in detail hereinafter.

The shape of the inorganic filler (C1) may be spherical or amorphous and is suitably selected together with its particle size. Known inorganic fillers may be used. For examples, the group I, II, III and IV metals of the periodic table, transition metals, oxides, hydroxides, chlorides, sulfates, sulfites, carbonates, phosphates and silicates thereof, and mixtures and composite salts of these may be used. More specifically, silicon dioxide, glass powders such as strontium glass, lanthanum glass and barium glass, quartz powders, barium sulfate powders, aluminum oxide powders, titanium oxide powders, barium salt powders, glass beads, glass fibers, barium fluoride powders, lead salt powders, glass powders containing talc, colloidal silica, silica gel, zirconium oxide powders, tin oxide powders and other ceramic powders may be used. Although the inorganic filler powders may be directly used in the dental restorative composition, to manufacture a cured product having excellent mechanical properties by increasing the amount of the inorganic filler, the inorganic filler powders are preferably made hydrophobic. Known surface treating agents may be used, as exemplified by dialkyldichlorosilanes such as γ-(meth)acryloxypropyl trimethoxysilane, vinyltriethoxysilane, 3-aminopropyltriethoxysilane, 3-chloropropyltrimethoxysilane silylisocyanate, vinyltrichlorosilane, dimethyldichlorosilane and dioctyldichlorosilane, silane coupling agents such as hexamethylene disilazane, and zirconium coupling agents and titanium coupling agents corresponding to the above coupling agents. As for the surface treating method, (a) the surface of the inorganic filler is treated with a surface treating agent alone by a ball mill, twin-cylinder mixer or Henschel mixer, (b) a surface treating agent diluted with an aqueous solution containing an organic solvent in which the organic solvent and water are uniformly mixed together, such as an ethanol aqueous solution is added to and mixed with the inorganic filler and then heated at 50 to 150°C for several minutes to several hours (dry method), (c) an inorganic filler is added to an organic solvent such as ethanol, toluene or xylene, an organic solvent containing a suitable amount of water or acid water for promoting hydrolysis and water to prepare slurry and then the above surface treating agent is added to the slurry to treat the surface of the inorganic filler at room temperature to reflux temperature for several minutes to several hours, the solvent is removed by a known method such as decantation or evaporation, and the obtained residue is heated at 50 to 150°C for several hours (wet method, slurry method), or (d) a surface treating agent or the above aqueous solution is directly sprayed over a high-temperature inorganic filler (spray method). Alternatively, (e) a surface treating agent is directly added to the polymerizable monomer (A) and an inorganic filler is blended with the resulting mixture (integral blending method). As a matter of course, commercially available inorganic fillers which have already been surface treated may be used directly or may be further surface treated by any one of the above methods. The optimum amount of the surface treating agent based on the inorganic filler may be determined from the specific surface area of the inorganic filler. In the case of an inorganic filler having an average particle diameter of 0.1 µm or more, the amount of the surface treating agent is preferably 0.1 to 20 parts by weight, more preferably 0.1 to 15 parts by weight, particularly preferably 0.1 to 10 parts by weight based on 100 parts by weight of the inorganic filler powders. The average particle diameter of the inorganic filler powders may be suitably selected in consideration of the performance of the dental restorative composition but preferably 0.001 to 100 µm, more preferably 0.001 to 30 µm, much more preferably 0. 001 to 10 µm, particularly preferably 0.001 to 5 µm. When the particle diameter falls within the particularly preferred range, the surface of the cured product becomes lustrous and the opposing tooth or the dental material is hardly damaged. Carbon fibers or polymer type fibers may be used as long as the color and mechanical properties of the dental restorative composition are not impaired.

Known organic powders may be used as the organic filler (C2) without restriction. Preferred examples of the organic filler include elastomer powders such as a homopolymer and copolymer of a (meth) acrylate, copolymer of a styrene-based monomer such as (meth)styrene or α-methylstyrene and butadiene, copolymer of acrylonitrile and butadiene, copolymer of acrylonitrile, butadiene and styrene, copolymer of an alkyl (meth)acrylate and a styrene-based monomer, copolymer of vinyl acetate, an alkyl (meth)acrylate and a styrene-based monomer, copolymer of an alkyl (meth)acrylate, a styrene-based monomer and a (meth) acrylate having at least one hydroxyl group in the molecule, and copolymer of styrene, an alkyl (meth)acrylate and butadiene. They may be used alone or in combination of two or more. When these organic filler powders are used, a cured product of the dental restorative composition becomes soft. Therefore, when the type and amount of the organic filler powders are suitably selected, tenacity is provided and cracking or chipping in the cavity of a mouth can be prevented. The average particle diameter of the organic filler (C2) may be suitably selected in consideration of the performance of the dental restorative composition but preferably 0.001 to 100 µm, more preferably 0.01 to 100 µm, much more preferably 0.1 to 50 µm.

The ratio of the above polyfunctional polymerizable compound (A), the polymerization initiator (B) and the filler (C) may be suitably determined from the sense of use of the dental restorative composition paste and the polishing properties, gloss and mechanical properties such as tenacity of its cured product. The amount of the component (B) is preferably 0. 001 to 10 parts by weight, more preferably 0. 005 to 10 parts by weight, much more preferably 0.01 to 5 parts by weight, particularly preferably 0.01 to 3 parts by weight based on 100 parts by weight of the component (A) . The amount of the component (C) is preferably 1 to 900 parts by weight, more preferably 10 to 800 parts by weight, much more preferably 50 to 750 parts by weight based on 100 parts by weight of the component (A). The inorganic filler (C1) and/or the composite filler (D) which will be described hereinafter are/is more preferred than the organic filler (C2) as the component (C) because mechanical strength and abrasion resistance become high.

The above dental restorative composition is powdered after polymerization to be used as a composite filler (D) for dental restorative compositions.

The method of manufacturing the composite filler (D) is not particularly limited, and known methods such as solution polymerization, suspension polymerization, emulsion polymerization and bulk polymerization may be employed, out of which bulk polymerization at an increased temperature under increased pressure shown below is preferred because the composite filler can be manufactured in a short period of time and the solvent is not necessary.

As a specific manufacturing method, a paste comprising the above polyfunctional compound (A), the thermopolymerization initiator (B2) such as benzoyl peroxide and the filler (C) is prepared by using an universal stirrer, Banbury mixer, double-screw roll or kneader, thermally cured by a heat compression molding machine at a pressure of 0.1 to 50 MPa and a temperature of 60 to 200°C for several minutes to several hours and ground by a mill such as a ball mill or jet mill until a desired average particle diameter or particle size distribution is obtained. A polymerizable monomer comprising the above polymerizable monomer having a functionality of 3 or more as the main component is preferred as the polyfunctional polymerizable compound (A), and inorganic oxide powders (C1) having an average particle diameter of 10 µm or less, specifically 1 µm or less are preferred and nano-sized inorganic oxide, specifically colloidal silica called "Aerosil" having an average particle diameter of 0.001 to 0.1 µm is particularly preferred as the filler (C). The amount of the filler (C) is preferably 1 to 900 parts by weight, more preferably 20 to 700 parts by weight, more preferably 70 to 500 parts by weight, particularly preferably 70 to 300 parts by weight based on 100 parts by weight of the polyfunctional polymerizable compound (A). The amount of the polymerization initiator (B) is preferably 0.005 to 10 parts by weight, more preferably 0.01 to 5 parts by weight, much more preferably 0.01 to 3 parts by weight based on 100 parts by weight of the polyfunctional polymerizable compound (A).

The average particle diameter of the composite filler (D) may be suitably selected in consideration of the performance of the dental restorative composition but preferably 0. 05 to 100 µm, more preferably 0.1 to 80 µm, much more preferably 1 to 70 µm, particularly preferably 1 to 50 µm. When the particle diameter falls within the particularly preferred range, the obtained cured product has excellent mechanical properties and a lustrous surface and the opposing tooth or the dental material is hardly damaged.

The composite filler (D) may be used directly or after the amount of a peroxide existent in the component (D) is reduced by adding a reducing agent or heating so as to improve the thermal stability of the dental restorative composition. When it is heated, if a polymerizable monomer (A2) having a nitrogen atom, an ether bond and an aromatic ring is contained, yellowing may occur. Therefore, the content of the component (A2) in the polyfunctional polymerizable compound (A) should be set to 50 wt% or less, preferably 30 wt% or less, more preferably 20 wt% or less. To improve affinity with the above component (A), the component (D) may be surface treated with the above surface treating agent such as a silane coupling agent, or treated with a polymerizable monomer having a functional group which may be hydrogen bonded or covalently bonded to the functional group of the composite particle, as exemplified by a polymerizable monomer having an epoxy group such as glycidyl (meth) acrylate, a polymerizable monomer having an cyclic ether group such as tetrahydrofurfuryl (meth)acrylate and a polymerizable monomer having a hydroxyl group such as pentaerythritol tri(meth)acrylate. Even when the composite particle does not have a functional group, a polymer obtained by polymerizing the residual double bond or peroxide in the composite with a polymerizable monomer may be used.

The dental restorative composition of the present invention containing the above composite filler (D) will be described hereinbelow.

The dental restorative composition of the present invention preferably comprises a polymerizable monomer (E), the above polymerization initiator (B) and the above composite filler (D).

A known polymerizable monomer may be preferably used as the polymerizable monomer (E), as exemplified by (meth)acrylate compounds listed above. Since a polymer derived from the component (A) which provides tenacity is used in the composite filler (D), the component (E) does not always have to contain the component (A).

A known polymerization initiator may be used as the polymerization initiator (B). A photopolymerization initiator (B1), a thermopolymerization initiator (B2) and a redox initiator (B3) are preferably used alone or in combination. When it is used in a paste such as a resin for dental crowns or resin inlay and the photopolymerization initiator (B1) is used, a resin structure can be cured at a desired timing after it is made.

The ratio of the above polymerizable monomer (E), the polymerization initiator (B) and the composite filler (D) may be suitably determined by evaluating the mechanical properties of a cured product of the obtained composition. The amount of the component (B) is preferably 0.001 to 10 parts by weight, more preferably 0.005 to 10 parts by weight, much more preferably 0.01 to 5 parts by weight, particularly preferably 0.01 to 3 parts by weight based on 100 parts by weight of the component (E). The amount of the component (D) is preferably 5 to 900 parts by weight, more preferably 10 to 800 parts by weight, much more preferably 50 to 750 parts by weight based on 100 parts by weight of the component (E). Due to this ratio, a cured product of a dental restorative material having excellent mechanical properties, especially tenacity can be obtained.

The composite filler (D) and the above filler (C) may be contained at the same time. The filler (C) may be suitably selected from the inorganic filler powders (C1), the organic filler powders (C2) and a composite filler (D1) containing no polymer of the polyfunctional polymerizable compound (A) containing a polyester chain. When the component (C2) is mainly used, the organic filler contained in a cured product of the dental restorative composition is selectively worn away, whereby the surface of the cured product becomes uneven and contamination in the cavity of a mouth is promoted disadvantageously. Then, the filler to be preferably combined with the composite filler (D) is the component (C1) and/or the component (D1).

The ratio of the above polymerizable monomer (E), the polymerization initiator (B), the filler (C) and the composite filler (D) may be suitably determined by evaluating the mechanical properties of a cured product. In order to improve the mechanical properties, especially tenacity of a cured product of the dental restorative composition, the amount of the component (B) is preferably 0.001 to 10 parts by weight, more preferably 0.005 to 10 parts by weight, much more preferably 0.01 to 5 parts by weight, particularly preferably 0.01 to 3 parts by weight based on 100 parts by weight of the component (E). The total amount of the components (C) and (D) is preferably 1 to 900 parts by weight, more preferably 10 to 800 parts by weight, much more preferably 50 to 750 parts by weight. The ratio of the filler (C) to the composite restorative material (D) may be suitably determined in consideration of the application purpose and operation ease of the composition and the mechanical properties, polishing properties and gloss of its cured product. To improve the mechanical properties, especially tenacity of a cured product of the dental restorative composition, the weight ratio of the composite filler (D) to the filler (C) is preferably 1 to 99:99 to 1, more preferably 5 to 95:95 to 5, much more preferably 20 to 80:80 to 20.

Other industrially well known components such as a stabilizer, ultraviolet light absorber, organic or inorganic pigment, dye, viscosity control agent, surface tension control agent, wetting aid, aggregate, and polymerizable monomer and polymer other than the above may be added to the restorative composition of the present invention.

The cured product of the dental restorative composition of the present invention has excellent mechanical properties such as tenacity, impact resistance and fracture resistance. When these properties are discussed in terms of bending properties, in a 3-point bending test in which a test specimen measuring 2 mm x 30 mm x 2 mm (thickness) is bent at 3 points at a span interval of 20 mm, a cross head speed of 1 mm/min and room temperature, as bending failure energy corresponding to the area of a stress-strain curve increases, its tenacity and impact resistance become more effective. Since the bending failure energy of the cured product is significantly improved when the dental restorative composition of the present invention is used, it is possible to form a structure which is hardly broken by the application of impact to the cured product.

The dental restorative composition of the present invention can be advantageously used not only in dental prosthetic materials and dental filling materials but also almost all kinds of resin-based materials including dental materials containing a filler and dental materials containing no filler such as dental adhesives, dental resin cements, resin modified glass ionomers, denture bases, cavity liners, cavity coating materials, hyperesthesis suppressing materials, dental manicures and Fischer sealants.

### Examples

The following examples are provided for the purpose of further illustrating the present invention but are in no way to be taken as limiting.

TMPT-3CL and D-TMP-4CL (manufactured by Shin Nakamura Kagaku Kogyo Co., Ltd.) were used as polyfunctional polymerizable compounds containing 3 or more polyester chains bonded to polymerizable groups in one molecule.
Structure of TMPT-3CL 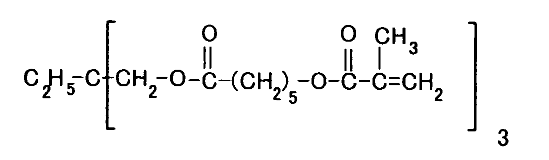 Structure of D-TMP-4CL

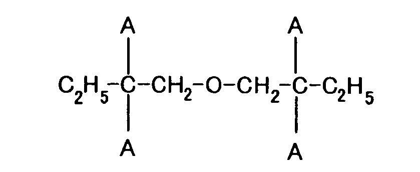

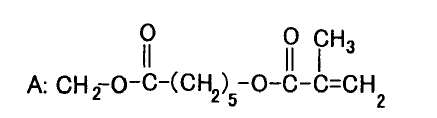

Trimethylolpropane trimethacrylate (TMPT) and ditrimethylolpropane tetramethacrylate (D-TMP) were used in Examples and Comparative Examples.

### Manufacturing Example 1

### (manufacture of TMPT-3CL composite filler (CF_{TMPT-3CL=100})

100 g of TMPT-3CL and 80 g of colloidal silica (average particle diameter of primary particles: 0.016 µm, R972 of Nippon Aerosil Co., Ltd.) were kneaded together by a kneader (BENCH KNEADER of Irie Shokai Co., Ltd.) to prepare a paste, and 0 .5 g of benzoyl peroxide was added to and further kneaded with the paste. This paste was thermally polymerized by a heat compression molding machine at a mold temperature of 120°C and a pressure of 5 to 10 MPa for 10 minutes. This cured product was dry ground by the LA-PO1 planetary ball mill (made of alumina) of ITOH Co., Ltd. for 30 minutes and classified by a #280-mesh sieve to collect composite filler powders. The average particle diameter of the composite filler powders was 20 µm.

### Manufacturing Example 2

### (manufacture of TMPT/TMPT-3CL = 75/25 composite filler (CF_{TMPT/TMPT-3CL=75/25}))

Composite filler powders (average particle diameter of 25 µm) were manufactured in the same manner as in Manufacturing Example 1 except that the total amount of TMPT-3CL was changed to TMPT-3CL (75 g) and TMPT (25 g).

### Manufacturing Example 3

### (manufacture of TMPT/TMPT-3CL = 50/50 composite filler (CF_{TMPT/TMPT-3CL=50/50}))

Composite filler powders (average particle diameter of 23 µm) were manufactured in the same manner as in Manufacturing Example 1 except that the total amount of TMPT-3CL was changed to TMPT-3CL (50 g) and TMPT (50 g).

### Manufacturing Example 4

### (manufacture of TMPT/TMPT-3CL = 25/75 composite filler (CFT_{MPT/TMPT-3CL=25/75}))

Composite filler powders (average particle diameter of 25 µm) were manufactured in the same manner as in Manufacturing Example 1 except that the total amount of TMPT-3CL was changed to TMPT-3CL (25 g) and TMPT (75 g).

### Manufacturing Example 5

### (manufacture of TMPT/TMPT-3CL = 10/90 composite filler (CF_{TMPT/TMPT-3CL=10/90}))

Composite filler powders (average particle diameter of 23 µm) were manufactured in the same manner as in Manufacturing Example 1 except that the total amount of TMPT-3CL was changed to TMPT-3CL (10 g) and TMPT (90 g).

### Manufacturing Example 6

### (manufacture of TMPT-3CL/UDMA = 90/10 composite filler (CF_{TMPT-3CL/UDMA=90/10}))

Composite filler powders (average particle diameter of 24 µm) were manufactured in the same manner as in Manufacturing Example 1 except that the total amount of TMPT-3CL was changed to TMPT-3CL (90 g) and di(methacryloxyethyl)trimethylhexamethylene diurethane (UDMA) (10 g).

### Manufacturing Example 7

### (manufacture of D-TMP-4CL composite filler (CF_{D-TMP-4CL=100}))

Composite filler powders (average particle diameter of 20 µm) were manufactured in the same manner as in Manufacturing Example 1 except that the total amount of TMPT-3CL was changed to D-TMP-4CL (100 g).

### Manufacturing Example 8

### (manufacture of D-TMPT-4CL/D-TMP = 25/75 composite filler (CF_{D-TMPT-4CL/D-TMP=75/25}))

Composite filler powders (average particle diameter of 23 µm) were manufactured in the same manner as in Manufacturing Example 1 except that the total amount of TMPT-3CL was changed to D-TMP-4CL (25 g) and D-TMP (75 g).

### Manufacturing Example 9

### (manufacture of TMPT composite filler (CF_{TMPT=100}))

Composite filler powders (average particle diameter of 22 µm) were manufactured in the same manner as in Manufacturing Example 1 except that the total amount of TMPT-3CL was changed to trimethylolpropane trimethacrylate (TMPT).

### Manufacturing Example 10

### (manufacture of D-TMP composite filler (CF_{D-TMP=100}))

Composite filler powders (average particle diameter of 21 µm) were manufactured in the same manner as in Manufacturing Example 1 except that the total amount of TMPT-3CL was changed to ditrimethylolpropane tetramethacrylate (D-TMP).

### Example 1

A photopolymerizable monomer composition (4.0 g) consisting of di(methacryloxyethyl)trimethylhexamethylene diurethane (UDMA), triethylene glycol dimethacrylate, camphorquinone and 2-n-butoxyethyl N,N-dimethylbenzoate in a weight ratio of 74.60/24.90/0.25/0.25 (wt%) was placed in a mortar under light shielding and kneaded with R972 (1.8 g) to prepare a paste which was then kneaded with the CFTMPT-3CL=100 composite filler (4.2 g) of Manufacturing Example 1 to prepare a paste. This paste was defoamed under vacuum to remove air bubbles contained in the paste and charged into a Teflon mold having a hole measuring 2 mm x 30 mm x 2 mm (thickness) beneath which a transparent glass plate was placed. After charging, a transparent glass plate was also placed on top of the Teflon mold and the paste was optically cured by a visible light illuminator (α-LightII of Morita Mfg. Co., Ltd.) for 3 minutes to manufacture a bending test specimen. After this test specimen was immersed in water at 37°C for 24 hours, a 3-point bending test was conducted by using the autograph (AGS-2000G) of Shimadzu Corporation at room temperature, a cross head speed of 1.0 mm/min and a span interval of 20 mm to investigate the bending characteristics of the test specimen. The results are shown in Table 1.

### Examples 2 to 8

Test specimens were manufactured in the same manner as in Example 1 except that the composite filler (CF_{TMPT-3CL}) was changed to the composite fillers of Manufacturing Examples 2 to 8 shown in Table 1 to investigate their bending characteristics.

### Comparative Examples 1 and 2

Test specimens were manufactured in the same manner as in Example 1 except that the composite filler (CF_{TMPT-3CL}) was changed to the composite fillers of Manufacturing Examples 9 and 10 to investigate their bending characteristics.

**Table 1**

| No. | Composite filler | bending strength (MPa) | bending failure energy (J) |
|---|---|---|---|
| Ex.1 | CF_{TMPT-3CL=100} | 79 | 0.041 |
| Ex.2 | CF_{TMPT/TMPT-3CL=75/25} | 91 | 0.025 |
| Ex.3 | CF_{TMPT/TMPT-3CL=50/50} | 80 | 0.023 |
| Ex.4 | CF_{TMPT/TMPT-3CL=25/75} | 81 | 0.026 |
| Ex.5 | CF_{TMPT/TMPT-3CL=10/90} | 75 | 0.031 |
| Ex.6 | CF_{TMPT-3CL/UDMA=90/10} | 79 | 0.030 |
| Ex.7 | CF_{D-TMP-4CL=100} | 78 | 0.033 |
| Ex.8 | CF_{D-TMPT-4CL/D-TMP=25/75} | 81 | 0.022 |
| C.Ex.1 | CF_{TMPT=100} | 85 | 0.014 |
| C.Ex.2 | CF_{D-TMP=100} | 84 | 0.015 |

When Examples 1 to 8 are compared with Comparative Examples 1 and 2, it is understood that the cured products of Examples 1 to 8 had excellent tenacity with bending failure energy which was 2 times or more larger than those of Comparative Examples though they were almost the same in bending strength.

### Example 9

A photopolymerizable monomer composition consisting of UDMA, TMPT-3CL, camphorquinone and 2-n-butoxyethyl N,N-dimethylaminobenzoate with a weight ratio of 89. 5/9. 9/0.3/0.3 (wt%) was charged into a Teflon mold having a hole measuring 2 mm x 30 mm x 2 mm (thickness) in which a transparent glass plate was placed. After charging, a transparent glass plate was also placed on top of the Teflon mold, and the composition was optically cured by a visible light illuminator (α-LightII of Morita Mfg. Co., Ltd.) for 3 minutes to manufacture a bending test specimen. After the test specimen was immersed in water at 37°C for 24 hours, a 3-point bending test was conducted by using the autograph (AGS-2000G) of Shimadzu Corporation at room temperature, a cross head speed of 1.0 mm/min and a span interval of 20 mm to investigate the bending characteristics of the specimen. The bending strength was 86 MPa and the bending failure energy was 0.078 J.

### Example 10

A test specimen was manufactured in the same manner as in Example 9 except that TMPT-3CL was changed to D-TMP-4CL to investigate its bending characteristics. The bending strength was 86 MPa and the bending failure energy was 0.086 J.

### Comparative Example 3

A test specimen was manufactured in the same manner as in Example 9 except that TMPT-3CL was changed to triethylene glycol dimethacrylate to investigate its bending characteristics. The bending strength was 90 MPa and the bending failure energy was 0.043 J. The bending strength was almost the same as those of Examples 9 and 10 but the bending failure energy was about 1/2 of those of Examples 9 and 10.

### Example 11

A photopolymerizable monomer composition (7.5 g) consisting of UDMA, TMPT-3CL, camphorquinone and 2-n-butoxyethyl N,N-dimethylaminobenzoate in a weight ratio of 89.5/9.9/0.3/0.3 (wt%) was placed in a mortar under light shielding and kneaded with R972 (2.5 g) to prepare a paste which was then defoamed under vacuum to remove air bubbles contained in the paste. The paste was charged into a Teflon mold having a hole measuring 2 mm x 30 mm x 2 mm (thickness) in which a transparent glass plate was placed. After charging, a transparent glass plate was also placed on top of the Teflon mold, and the composition was optically cured by a visible light illuminator (α-LightII of Morita Mfg. Co. , Ltd.) for 3 minutes to manufacture a bending test specimen. After the test specimen was immersed in water at 37°C for 24 hours, a 3-point bending test was conducted by using the autograph (AGS-2000G) of Shimadzu Corporation at room temperature, a cross head speed of 1.0 mm/min and a span interval of 20 mm to investigate the bending characteristics of the specimen. The bending strength was 111 MPa and the bending failure energy was 0.082 J.

### Example 12

A paste was manufactured in the same manner as in Example 11 except that TMPT-3CL was changed to D-TMP-4CL to conduct a bending test. The bending strength was 116 MPa and the bending failure energy was 0.076 J.

### Comparative Example 4

A photopolymerizable monomer composition (7.5 g) consisting of UDMA, triethylene glycol dimethacrylate, camphorquinone and 2-n-butoxyethyl N,N-dimethylaminobenzoate in a weight ratio of 89.5/9.9/0.3/0.3 (wt%) was placed in a mortar under light shielding and kneaded with R972 (2.5 g) to prepare a paste which was then defoamed under vacuum to remove air bubbles contained in the paste. It was charged into a Teflon mold having a hole measuring 2 mm x 30 mm x 2 mm (thickness) beneath which a transparent glass plate was placed. After charging, a transparent glass plate was also placed on top of the Teflon mold, and the composition was optically cured by a visible light illuminator (α-LightII of Morita Mfg. Co., Ltd.) for 3 minutes to manufacture a bending test specimen. After the test specimen was immersed in water at 37°C for 24 hours, a 3-point bending test was conducted by using the autograph (AGS-2000G) of Shimadzu Corporation at room temperature, a cross head speed of 1.0 mm/min and a span interval of 20 mm to investigate the bending characteristics of the specimen.

The bending strength was 98 MPa and the bending failure energy was 0.048 J. The bending strength was about 10 MPa lower than those of Examples 11 and 12 and the bending failure energy was about 1/2 of those of Examples 11 and 12.

## Claims

1. A dental restorative composition comprising a polyfunctional polymerizable compound (A) having at least 3 partial chains in one molecule, each having a polymerizable group, an ester bond and a length of at least 7 atoms.

2. The composition according to claim 1, wherein the partial chain has a length of at least 9 atoms.

3. The composition according to claim 1, wherein the partial chain is a polyester chains.

4. The composition according to claim 1, wherein the particle chain is represented by the following formula (I): 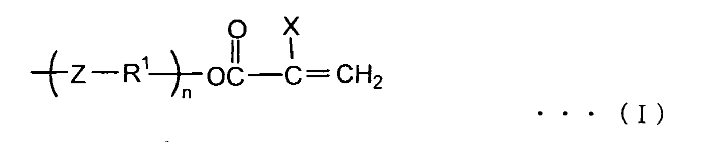 wherein R¹ is an alkylene group having 1 to 10 carbon atoms or a divalent group having an aromatic ring and/or a hetero ring, Z is -OCO- or -COO-, X is a hydrogen atom, halogen atom, cyano group, alkyl group having 1 to 10 carbon atoms or monovalent group having an aromatic ring and/or a hetero ring, and n is an integer of 1 to 10.

5. The composition according to claim 1, wherein the polyfunctional polymerizable compound (A) is at least one selected from the group consisting of compounds represented by the following formulas (II) and (III): 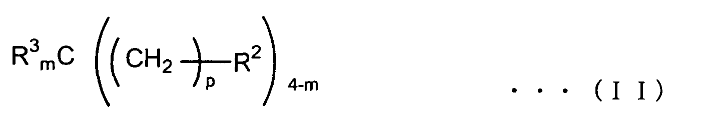 wherein R² is a partial chain represented by the above formula (I), R³ is a hydrogen atom, alkyl group having 1 to 10 carbon atoms, monovalent group having an aromatic ring and/or a hetero ring or group containing a polymerizable group, p is an integer of 0 to 10, and m is 0 or 1, 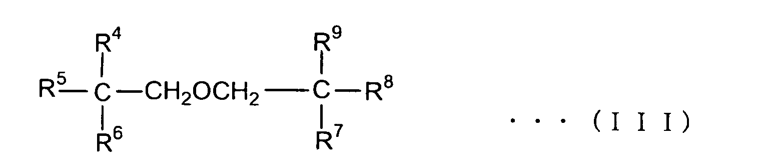 wherein R⁴, R⁵, R⁶, R⁷, R⁸ and R⁹ are each independently a group represented by the following formula, 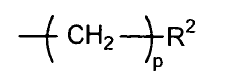 hydroxyl group, alkyl group having 1 to 10 carbon atoms, hydroxylalkyl group having 1 to 10 carbon atoms, monovalent group having an aromatic ring and/or a hetero ring, or monovalent group having a polymerizable group which differs from the group represented by the following formula: 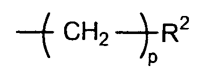 with the proviso that at least 3 out of R⁴ to R⁹ are each a group represented by the following formula (R² and p are as defined in the above formula (II)):

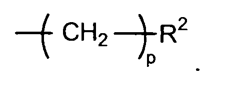

6. The composition according to any one of claims 1 to 5 which further comprises (B) a polymerization initiator.

7. The composition according to claim 6 which comprises the polymerization initiator (B) in an amount of 0.005 to 10 parts by weight based on 100 parts by weight of the polyfunctional polymerizable compound (A).

8. The composition according to any one of claims 1 to 7 which further comprises (C) a filler.

9. The composition according to claim 8, wherein the average particle diameter of the filler (C) is 0.001 to 100 µm.

10. The composition according to claim 8 or 9 which comprises the polymerization initiator (B) in an amount of 0.005 to 10 parts by weight and the filler (C) in an amount of 1 to 900 parts by weight based on 100 parts by weight of the polyfunctional polymerizable compound (A).

11. The composition according to any one of claims 1 to 10 which further comprises (D) a composite filler which is obtained by grinding a cured product formed by polymerizing the composition of any one of claims 1 to 10.

12. The composition according to claim 11, wherein the average particle diameter of the composite filler (D) is 0.05 to 100 µm.

13. The composition according to claim 11 or 12 which further comprises (E) a polymerizable monomer which differs from the above polyfunctional polymerizable compound (A) and the polymerization initiator (B).

14. The composition according to claim 13 which comprises the polymerization initiator (B) in an amount of 0.01 to 10 parts by weight and the composite filler (D) in an amount of 5 to 900 parts by weight based on 100 parts by weight of the polymerizable monomer (E).

15. The composition according to any one of claims 11 to 14 which further comprises the filler (C).

16. The composition according to claim 15 which comprises the polymerization initiator (B) in an amount of 0.005 to 10 parts by weight and the filler (C) and the composite filler (D) in a total amount of 10 to 900 parts by weight based on 100 parts by weight of the polymerizable monomer (E).

17. The composition according to claim 15 or 16, wherein the weight ratio of the filler (C) to the composite filler (D) is 1:99 to 99:1.

## Amended claims

### Amended claims under Art. 19.1 PCT

**1.** (Amended) A dental restorative composition comprising
(D) a composite filler which is obtained by grinding a cured product formed by polymerizing a composition containing a polyfunctional polymerizable compound (A) having at least 3 partial chains in one molecule, each having a polymerizable group, an ester bond and a length of at least 7 atoms, and the polyfunctional polymerizable compound (A) having at least 3 partial chains in one molecule, each having a polymerizable group, an ester bond and a length of at least 7 atoms.

**2.** (Amended) The dental restorative composition according to claim 1, wherein the partial chain of the polyfunctional polymerizable compound (A) has a length of at least 9 atoms.

**3.** (Amended) The dental restorative composition according to claim 1, wherein the partial chain of the polyfunctional polymerizable compound (A) is a polyester chain.

**4.** (Amended) The dental restorative composition according to claim 1, wherein the particle chain of the polyfunctional polymerizable compound (A) is represented by the following formula (I): 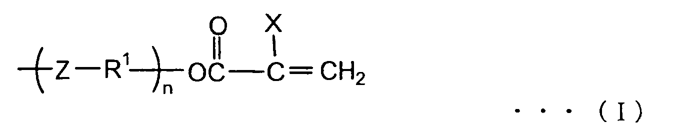 wherein R¹ is an alkylene group having 1 to 10 carbon atoms or a divalent group having an aromatic ring and/or a hetero ring, Z is -OCO- or -COO-, X is a hydrogen atom, halogen atom, cyano group, alkyl group having 1 to 10 carbon atoms or monovalent group having an aromatic ring and/or a hetero ring, and n is an integer of 1 to 10.

**5.** (Amended) The dental restorative composition according to claim 1, wherein the polyfunctional polymerizable compound (A) is at least one selected from the group consisting of compounds represented by the following formulas (II) and (III): 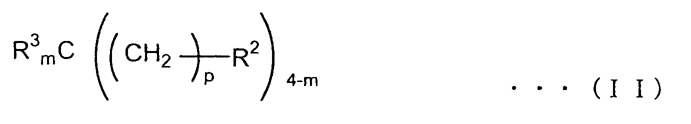 wherein R² is a partial chain represented by the above formula (I), R³ is a hydrogen atom, alkyl group having 1 to 10 carbon atoms, monovalent group having an aromatic ring and/or a hetero ring or group containing a polymerizable group, p is an integer of 0 to 10, and m is 0 or 1, 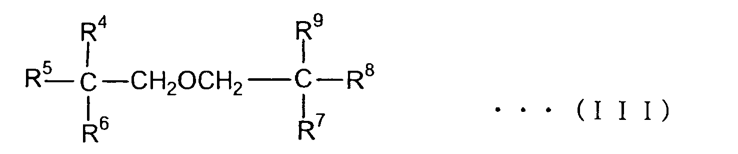 wherein R⁴, R⁵, R⁶, R⁷, R³ and R⁹ are each independently a group represented by the following formula, 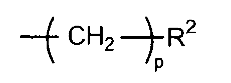 hydroxyl group, alkyl group having 1 to 10 carbon atoms, hydroxylalkyl group having 1 to 10 carbon atoms, monovalent group having an aromatic ring and/or a hetero ring, or monovalent group having a polymerizable group which differs from the group represented by the following formula: 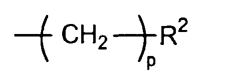 with the proviso that at least 3 out of R⁴ to R⁹ are each a group represented by the following formula (R² and p are as defined in the above formula (II)):

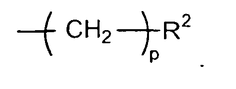

**6.** (Amended) The dental restorative composition according to any one of claims 1 to 5 which further comprises (B) a polymerization initiator.

**7.** (Amended) The dental restorative composition according to claim 6 which comprises the polymerization initiator (B) in an amount of 0.005 to 10 parts by weight based on 100 parts by weight of the polyfunctional polymerizable compound (A).

**8.** (Amended) The dental restorative composition according to any one of claims 1 to 7 which further comprises (C) a filler.

**9.** (Amended) The dental restorative composition according to claim 8, wherein the average particle diameter of the filler (C) is 0.001 to 100 µm.

**10.** (Amended) The dental restorative composition according to claim 8 or 9 which comprises the polymerization initiator (B) in an amount of 0.005 to 10 parts by weight and the filler (C) in an amount of 1 to 900 parts by weight based on 100 parts by weight of the polyfunctional polymerizable compound (A).

**11.** (Cancelled)

**12.** (Amended) The dental restorative composition according to claim 1, wherein the average particle diameter of the composite filler (D) is 0.05 to 100 µm.

**13.** (Amended) The dental restorative composition according to claim 1, wherein the composition for the composite filler (D) further comprises (E) a polymerizable monomer which differs from the above polyfunctional polymerizable compound (A) and the polymerization initiator (B) .

**14.** (Amended) The dental restorative composition according to claim 13 which comprises the polymerization initiator (B) in an amount of 0.01 to 10 parts by weight and the composite filler (D) in an amount of 5 to 900 parts by weight based on 100 parts by weight of the polymerizable monomer (E).

**15.** (Amended) The dental restorative composition according to any one of claims 12 to 14 which further comprises the filler (C).

**16.** (Amended) The dental restorative composition according to claim 15 which comprises the polymerization initiator (B) in an amount of 0.005 to 10 parts by weight and the filler (C) and the composite filler (D) in a total amount of 10 to 900 parts by weight based on 100 parts by weight of the polymerizable monomer (E).

**17.** (Amended) The dental restorative composition according to claim 15 or 16, wherein the weight ratio of the filler (C) to the composite filler (D) is 1:99 to 99:1.

**18.** (Added) The dental restorative composition according to claim 1, wherein the composite filler (D) further comprises an inorganic oxide powder (C1) having an average diameter of 1 µm or less in an amount of 1 to 900 parts by weight based on 100 parts by weight of the polyfunctional polymerizable compound (A).
